# EUROPEAN PATENT APPLICATION

(11) **EP 4 249 509 A1**
(43) Date of publication of application: **27.09.2023**
(21) Application number: 22305337.2
(22) Date of filing: 22.03.2022
(51) Int. Cl.: C07K 16/22, A61P 19/02, A61K 39/395

(54) **ANTI-NETRIN-1 ANTIBODY AGAINST ARTHRITIS-ASSOCIATED PAIN**

(71) Applicant: Netris Pharma, 69008 Lyon (FR)
(72) Inventor: SVENSSON, Camilla, 11418 STOCKHOLM (SE); RUDJITO, Resti, 75644 UPPSALA (SE); NEVES, David, 69390 VERNAISON (FR)
(74) Representative: Lavoix

(57) **Abstract**

The present invention is related to the use of an anti-netrin-1 antibody or an antigen-binding fragment thereof, as an analgesic or antinociceptive medicament, in particular in the context of an arthritis-associated chronic pain, such as Rheumatoid arthritis-associated chronic pain. Humanized monoclonal antibodies are disclosed. The invention also relates to a method for prevention or treatment of pain.

## Description

The present invention is related to novel analgesic or antinociceptive agents, or pain-relieving agents, useful against arthritis-associated or arthritis-related pain, especially in rheumatoid arthritis (RA), allowing treating arthritis pain in a mammal, in particular a human patient in need thereof. In particular, these agents are beneficial during the period the patients have reduced disease activity but remaining pain. These agents are antibodies or antigen-binding fragments thereof, specifically binding to netrin-1. The invention also relates to a method for prevention or treatment of pain.

### Background of the invention

While numerous analgesic or antinociceptive medicaments are currently available, the adequate relief of pain remains an unmet medical need for many acute and chronic pain states.

Rheumatoid arthritis (RA) is a chronic autoimmune disease, typically presented as symmetrical pathology in small synovial joints including synovitis, cartilage serration and bone erosion. It is accompanied by symptoms such as joint pain, swelling and fatigue that may have debilitating effects on the functional ability and quality of life. The experience of pain for individuals with RA is highly varied, not only in terms of intensity, but also quality. Patients with RA often describe pain as local joint ache that may indicate a direct consequence of joint pathology and/or burning pain that is more characteristic of neuropathic pain. Widespread pain with associated sleep disturbances and low mood is also frequently reported. Furthermore, joint pain often develops before visual signs of inflammation and many patients report pain even after their disease activity is well controlled via pharmacological interventions.

Rheumatoid arthritis is thus often characterized by eroded joints and chronic pain that outlasts disease activity. Whilst several reports show strong associations between bone resorption and nociception, the underlying mechanisms remain to be unraveled.

While the inflamed synovium has traditionally been considered as the sole elicitor of pain in RA, the frequent misalignment between inflammation and pain has encouraged researchers to search for additional mechanisms that drive pain in arthritis. The notion that structural changes in bone such as focal and subchondral bone loss are associated with pain has underbuilt the hypothesis that osteoclasts, the cells that degrade bone, play a prominent role in the regulation of processes leading to sustained pain. Indeed, osteoclasts can induce nociception and several preclinical and clinical studies have demonstrated the benefits of osteoclast inhibitors, including bisphosphonates and calcitonin, in alleviating hyperalgesia in multiple bone disorders.

### Summary of invention

A collagen antibody-induced arthritis (CAIA) model was generated. Surprisingly, it was observed that Netrin-1 has pronociceptive properties in this model, with a direct effect of netrin-1 on nociceptors, especially during the chronic or late pain phase. Still unexpectedly, CAIA-induced hypersensitivity in the late phase was reversed by an anti-netrin-1 antibody. Systemic administration of the anti-netrin-1 antibody (NP137, 10 mg/kg, every second day) did not affect arthritis score or mechanical hypersensitivity in the inflammatory phase. However a progressive reversal of CAIA-induced hypersensitivity was observed in the late phase with NP137. To further confirm the pronociceptive property of netrin-1, *in vitro* calcium imaging experiments with primary dorsal root ganglia (DRG) neurons were performed. Netrin-1 stimulation of primary DRG neurons increased the percentage of cells that showed elevated intracellular Ca²⁺ signal in a dose-dependent fashion. We here identified the possible nociceptive roles of netrin-1 released by osteoclasts. While the exact mechanisms as to how these factors induce pain in the CAIA model remains unclear, these findings unexpectedly show that targeting netrin-1 was sufficient to produce antinociceptive effects and thus that anti-netrin-1 antibodies can be used as an analgesic or antinociceptive medicament, especially in arthritis in the late phase.

In the rheumatoid arthritis (RA) model, there is a period of joint inflammation that lasts for 3-4 weeks. The mice are very sensitive during this time. Despite the joint inflammation goes away at some point, the signs of pain do not resolve in the mice. The mice display signs of hypersensitivity to stimulation (pain) for a long time, an additional 2-3 months, even though signs of inflammation are not seen. The present data suggest that the pain become chronic in part due to sprouting of the nerve fibers in the bone and other tissues in the joints, and in part because of changes in the spinal cord. These type of models of RA have received a lot of attention as this is an emergent problem in RA patients. With the new drugs efficiently treating the inflammation and disease activity one expected the pain to go away as well, but, studies report that 10-50% of the patients that have low or no disease activity still have clinically unaccepted levels of pain. The present data show that netrin-1 inhibition resolve pain in the mice in this "late" or "post-inflammatory" phase of the model, and not during the phase of joint inflammation. NP137 also do not reduce the degree of inflammation in the present model.

In an aspect, the invention relates to an anti-netrin-1 antibody or an antigen-binding fragment thereof, or a pharmaceutical composition containing an anti-netrin-1 antibody or an antigen-binding fragment thereof, and a pharmaceutically acceptable vehicle, for use as an analgesic or antinociceptive medicament or a pain-relieving medicament, to treat arthritis-associated pain.

In particular, the arthritis concerned by the invention are those wherein pain is associated with netrin-1. In an embodiment, arthritis is RA and the antibody is used to treat Rheumatoid arthritis-associated pain.

The present use preferably allows pain relief, attenuation or elimination of pain.

In an aspect, the invention relates to an anti-netrin-1 antibody or an antigen-binding fragment thereof, or a pharmaceutical composition containing an anti-netrin-1 antibody or an antigen-binding fragment thereof, and a pharmaceutically acceptable vehicle, for use as an analgesic or antinociceptive medicament to treat post-inflammation or chronic phase arthritis-associated pain, in particular post-inflammation or chronic Rheumatoid arthritis-associated pain. This use preferably allow pain relief, attenuation or elimination of pain.

In an aspect, the invention relates to an anti-netrin-1 antibody or an antigen-binding fragment thereof, or a pharmaceutical composition containing an anti-netrin-1 antibody or an antigen-binding fragment thereof, and a pharmaceutically acceptable vehicle, for use in treating Rheumatoid arthritis pain. This use preferably allows pain relief, attenuation or elimination of pain.

In an aspect, the invention relates to a method for treatment of arthritis-associated pain, in particular Rheumatoid arthritis-associated pain, comprising administering, to a subject in need thereof, an effective amount of an anti-netrin-1 antibody or an antigen-binding fragment thereof, or a pharmaceutical composition containing an anti-netrin-1 antibody or an antigen-binding fragment thereof, and a pharmaceutically acceptable vehicle. In an aspect, the method is to treat post-inflammation or chronic arthritis-associated pain, in particular post-inflammation or chronic Rheumatoid arthritis-associated pain, in a subject having such a pain. Generally, these are patients reporting pain even after their disease activity is well controlled via pharmacological interventions. The method preferably allows pain relief, attenuation or elimination of pain.

In an aspect, the invention relates to the use of an anti-netrin-1 antibody or an antigen-binding fragment thereof, or a pharmaceutical composition containing an anti-netrin-1 antibody or an antigen-binding fragment thereof, and a pharmaceutically acceptable vehicle, for the manufacture of an analgesic or antinociceptive medicament, especially for arthritis-associated pain, in particular Rheumatoid arthritis-associated pain. In an aspect, this pain is post-inflammation or chronic arthritis-associated pain, in particular post-inflammation or or chronic Rheumatoid arthritis-associated pain.

In an aspect, these uses and methods of use allow pain relief, attenuation or elimination of pain caused or potentiated by the activation of the nociceptive system by netrin-1.

In an aspect, the anti-netrin-1 antibody exerts an antinociceptive effect, leading to pain relief or elimination of pain. In response to noxious stimuli, nociceptors transmit signals to central nervous system neurons in neural pathways associated with pain sensation. Without willing being bound to theory, it is deemed that the antibody, by capturing or interfering with netrin-1, induces an attenuation or an inhibition of the response of nociceptors to noxious stimuli.

### Detailed description

The antibody may be a polyclonal or monoclonal antibody specifically binding to netrin-1 (NTN1) (anti-netrin-1 antibody or antibody binding to netrin-1), especially human netrin-1.

An anti-netrin-1 polyclonal antibody may, inter alia, be obtained by immunizing an animal such as a rabbit, a mouse and the like with the aid of the selected amino acid sequence, collecting and then depleting the antiserum obtained on, for example, an immunoadsorbent containing the receptor according to methods known per se to a person skilled in the art.

The netrin-1 amino acid sequence is as depicted on SEQ ID NO: 1 and netrin-1 may be used in whole or in part to generate polyclonal or monoclonal antibodies.

Generally, monoclonal antibodies may be obtained according to the conventional method of lymphocyte fusion and hybridoma culture described by Köhler and Milstein, (Nature, 1975, 256(5517): 495-7). Other methods for preparing monoclonal antibodies are also known (Harlow et al., ed., 1988 "Antibodies: a laboratory manual"). The monoclonal antibodies may be prepared by immunizing a mammal (for example a mouse, a rat, a rabbit or even a human being, and the like) and using the lymphocyte fusion technique leading to hybridoma (Köhler and Milstein, 1975). Alternative techniques to this customary technique exist. It is possible, for example, to produce monoclonal antibodies by expressing a nucleic acid cloned from a hybridoma. It is also possible to produce antibodies by the phage display technique by introducing cDNAs for antibodies into vectors, which are typically filamentous phages which exhibit gene libraries V at the surface of the phage (for example fUSE5 for *E*. *coli,* Scott J.K., Smith G.P. Science 1990; 249:386-390). Protocols for constructing these antibody libraries are described in J.D. Marks et al., J. Mol. Biol., 222 (1991), p. 581). The cDNA corresponding to full length netrin-1 with signal sequence (SEQ ID NO: 2) or to a suitable fragment thereof may be used to produce monoclonal antibodies according to these methods.

The anti-netrin-1 monoclonal antibody (mAb) may be a murine, a chimeric, a humanized or a full-human monoclonal antibody. The fragment may be any type of mAb fragment that keeps substantially the ability of the whole antibody to bind to Netrin-1, it can be for example a Fab or a F(ab')₂. In particular, a monoclonal antibody is one disclosed in WO2015/104360 or US10,494,427, which documents are incorporated herein by reference, and disclose useful murine, chimeric and humanized monoclonal antibodies. These are antibodies or fragments thereof, which specifically bind to a netrin-1 epitope or to a polypeptide having the amino acid sequence SEQ ID NO: 3 or 33, or a variant thereof.

The antibodies useful in the invention may be defined by their CDRs. In particular these CDRs are derived from the murine antibody 4C11 disclosed in WO2015/104360 or US10,494,427, which antibody specifically binds to the polypeptide having the amino acid sequence SEQ ID NO: 3 or 33. Preferably, the antibody is a monoclonal antibody or an antigen-binding fragment thereof, comprising
a variable domain VH comprising:
   - a H-CDR1 having a sequence set forth as SEQ ID NO: 5;
   - a H-CDR2 having a sequence set forth as SEQ ID NO: 6;
   - a H-CDR3 having a sequence set forth as SEQ ID NO: 7;
a variable domain VL comprising:
   - a L-CDR1 having a sequence set forth as SEQ ID NO: 8;
   - a L-CDR2 having a sequence YAS;
   - a L-CDR3 having a sequence set forth as SEQ ID NO: 9; or
a variable domain VH comprising:
   - a H-CDR1 having a sequence set forth as SEQ ID NO: 28;
   - a H-CDR2 having a sequence set forth as SEQ ID NO: 29;
   - a H-CDR3 having a sequence set forth as SEQ ID NO: 30;
a variable domain VL comprising:
   - a L-CDR1 having a sequence set forth as SEQ ID NO: 31;
   - a L-CDR2 having a sequence set forth as SEQ ID NO: 32;
   - a L-CDR3 having a sequence set forth as SEQ ID NO: 9.

In a first series of embodiments, the antibody of the invention comprises an amino acid sequence SEQ ID NO: 10, 11, 12 or 13. Typically, it comprises a VH of sequence SEQ ID NO: 10 and a VL of sequence SEQ ID NO: 11, or a heavy chain of sequence SEQ ID NO: 12 and a light chain of sequence SEQ ID NO: 13.

In a second series of embodiments, the antibody is chimeric. Preferably, it comprises a VH of sequence SEQ ID NO: 27 and a VL of sequence SEQ ID NO: 19.

In a third series of embodiments, the antibody is humanized. Preferably, it comprises an amino acid sequence selected from the group of SEQ ID NO: 14 to 18 (VL) and/or from the group of SEQ ID NO: 20 to 26 (VH). Typically, the antibody is humanized and comprises a VH having an amino acid sequence selected from the group of SEQ ID NO: 14 to 18 and a VL having an amino acid sequence selected from the group of SEQ ID NO: 20 to 26.

The antibody preferably comprises a monoclonal antibody (mAb) or an antigen-binding fragment thereof, wherein the mAb or its fragment specifically binds to Netrin-1. The mAb may be a murine, a chimeric, a humanized or a full-human monoclonal antibody. The fragment may be any type of mAb fragment that keeps substantially the ability of the whole antibody to bind to Netrin-1, it can be for example a Fab or a F(ab')₂.

Specific embodiments disclosed in this prior document and that can be used herein are the following antibodies listed in Table 1. The first listed antibody is a chimeric 4C11 antibody, comprising the murine VH and VL of the murine 4C11 antibody. HUM00 listed in Table 1 corresponds to the grafting of the murine 4C11 CDRs into a human IgG1. The ten humanized mAb HUM01 to HUM10 correspond to humanized mAbs derived from HUM00 with the same CDRs, but specific modifications in the FR regions of the human IgG. HUM03 is also publicly known as NP137 and is currently under clinical trials. Sequences of the human IgG1 CH come from Genbank AEL33691.1 modified R97K. Sequences of the human IgG1 CL (Kappa) come from Genbank CAC20459.1. The other allotypes may be used as well. Specific binding of all these mAbs, murine, chimeric and humanized HUM01-HUM10, Fab fragments and F(ab')₂ fragments, to Netrin-1 and their ability to inhibit binding of netrin-1 to its receptor UNC5B, are demonstrated in US 10,494,427 (Example 3).

In particular these antibodies specifically bind to the polypeptide having the amino acid sequence SEQ ID NO: 33.

| Table 1 | VH SEQ ID NO: | Constant heavy chain | VL SEQ ID NO: | Constant light chain |
|---|---|---|---|---|
| 4C11 | 10 | Human IgG1 | 11 | Human IgG1 |
| HUM00 | 27 | Human IgG1 | 19 | Human IgG1 |
| HUM01 | 20 | Human IgG1 | 14 | Human IgG1 |
| HUM02 | 21 | Human IgG1 | 15 | Human IgG1 |
| HUM03 | 22 | Human IgG1 | 16 | Human IgG1 |
| HUM04 | 23 | Human IgG1 | 17 | Human IgG1 |
| HUM05 | 24 | Human IgG1 | 17 | Human IgG1 |
| HUM06 | 25 | Human IgG1 | 16 | Human IgG1 |
| HUM07 | 26 | Human IgG1 | 17 | Human IgG1 |
| HUM08 | 22 | Human IgG1 | 17 | Human IgG1 |
| HUM09 | 25 | Human IgG1 | 18 | Human IgG1 |
| HUM10 | 21 | Human IgG1 | 16 | Human IgG1 |

Preferably, the antibody is a monoclonal antibody or an antigen-binding fragment thereof, comprising a pair of VH and VL sequences selected from the following pairs: SEQ ID NO: 27 and 19, SEQ ID NO: 20 and 14, SEQ ID NO: 21 and 15, SEQ ID NO: 22 and 16, SEQ ID NO: 23 and 17, SEQ ID NO: 24 and 17, SEQ ID NO: 25 and 16, SEQ ID NO: 26 and 17, SEQ ID NO: 22 and 17, SEQ ID NO: 25 and 18, SEQ ID NO: 21 and 16. More preferably, the antibody is a monoclonal antibody or an antigen-binding fragment thereof, comprising a pair of VH and VL sequences SEQ ID NO: 22 and 16.

The anti-netrin-1 antibody may further comprise a Human IgG1 Constant heavy chain (CH) and/or a Human IgG1 Constant light chain (CL), in particular a human kappa constant domain.

In an embodiment, sequences of the human IgG1 CH come from Genbank AEL33691.1 modified R97K. Sequences of the human IgG1 CL (Kappa) come from Genbank CAC20459.1. In an embodiment, the mAb is NP137 (AB_2811180 in The Antibody Registry) and comprises SEQ ID NO: 22 and 16 as VH, respectively VL sequences, and those specific IgG1 CH and CL.

The term "antigen-binding fragment" of an antibody (or "antibody-binding portion"), as used herein, refers to one or more fragments of an antibody that retain the ability to specifically bind to netrin-1. An antibody fragment may include, for example, a Fab fragment, a F(ab')₂ fragment, a Fv fragment, a dAb fragment, a fragment containing a CDR, or an isolated CDR. In an aspect, the fragment comprises the VH and VL sequences of an antibody selected from HUM00 to HUM10.

As anti-netrin-1 antibodies that may be used, one may cite other antibodies, especially monoclonal antibodies, or their antigen-binding fragments, developed against human netrin-1 or against animal netrin-1, netrin-1 being very homologous among species. May be cited: Abcam antibodies ab126729, ab122903, ab201324, ab39370; AF1109, AF6419, AF128.

In one embodiment, an antibody or antigen-binding fragment thereof having applications in a use or method of the present invention is administered as a single dose, or an initial dose followed by administration of a second or a plurality of subsequent doses of the antibody or antigen-binding fragment thereof, wherein the subsequent doses are separated by at least one day; at least one week, at least 2 weeks; at least 3 weeks; at least 4 weeks; at least 5 weeks; at least 6 weeks; at least 7 weeks; at least 8 weeks; at least 9 weeks; at least 10 weeks; at least 12 weeks; or at least 14 weeks. The dose may vary depending upon the age and the weight of a subject to be administered, target disease, conditions, route of administration, and the like. When the antibody of the present invention is used for treating pain in an adult patient, it is advantageous to administer the antibody of the present invention either intravenously or subcutaneously. One single dose or the subsequent doses may comprise an amount of antibody or fragment thereof of at least 0.1 or 1 mg/kg body weight, e.g. about 1 mg/kg body weight to about 100 mg/kg body weight, in particular about 10 mg/kg body weight to about 60 mg/kg body weight of the subject. Depending on the severity of the chronic pain, the frequency and the duration of the treatment can be adjusted.

### Definitions and further embodiments, variants and alternatives of the invention:

Throughout the instant application, the term "comprising" is to be interpreted as encompassing all specifically mentioned features as well optional, additional, unspecified ones. As used herein, the use of the term "comprising" also discloses the embodiment wherein no features other than the specifically mentioned features are present (*i.e.* "consisting of").

In the context of the invention, the term "treating" or "treatment", as used herein, means alleviating or inhibiting pain.

According to the invention, the term "patient" or "subject" is used interchangeably and is intended for a human or non-human mammal affected or likely to be affected with arthritis pain, in particular Rhumatoid arthritis pain. As recited above, this pain will be mostly chronic or post-inflammatory pain.

"Arthritis-associated pain" means pain caused by Arthritis. It is also called "arthritis-related pain". In these arthritis concerned by the invention, pain is associated at least with netrin-1, meaning that netrin-1 is present at a time in the process leading to pain generation, or, even, is directly or closely involved in the generation of pain (for example, in RA, it is deemed netrin-1 has a direct effect on nociceptors), especially during the chronic or late pain phase Of interest, pain can be felt during long period even when the inflammation or the arthritis disease activity is resolved: this is the so-called chronic or post-inflammatory arthritis pain.

Usual rheumatoid arthritis classification criteria such as DAS28 and ACR/EULAR 2010 (J. Kay and K.S. Upchurch, Rheumatology 2012: 51: vi5-vi9) may be used to identify a patient being at this state of chronic pain. DAS28 is a measure of disease activity in rheumatoid arthritis (RA). DAS stands for « disease activity score ».

It is also possible to make this identification by measuring markers correlated with inflammation or any other event, in RA patients. Patients having both pain and a marker level different from the normal values in the inflammatory phase, may be considered to be in the low or no disease activity phase. As an example, the C-reactive protein (CRP) level may be used, and, in particular, a blood CRP level < 10 mg/liter corresponds to a low or no inflammatory level. A patient having pain and such a CRP level is qualified to be treated by the invention. Blood CRP level is commonly measured in hospitals and laboratories, especially using automated clinical chemistry analyzers. It is a standard test and it can for example be an antibody-based method.

Another method is the visual analogue scale (from Wolfe F, Michaud K. Assessment of pain in rheumatoidarthritis: minimal clinically si gnificant difference, predictors, and the effect of anti-tumor necrosis factor therapy. J Rheumatol 2007;34:1674-83 ; and Atlavil et al., Arthritis Care Res (Hoboken) 2016 Aug;68(8):1061-8. doi: 10.1002/acr.22790). Pain more than 20 mm on the scale makes the patient qualified for treatment.

Individuals having chronic pain and treatable in accordance with the invention may be those having lowered DAS28 (lowered with respect to disease activity in rheumatoid arthritis), low CRP for a clinician, in particular a CRP equal or below 10 mg/liter, and/or a visual analogue scale equal or more than 20mm.

By a "therapeutically effective amount" of the antibody of the invention is meant a sufficient amount thereof to treat said pain, at a reasonable benefit/risk ratio applicable to any medical treatment. It will be understood, however, that the total daily usage of the antibody on will be decided by the attending physician within the scope of sound medical judgment. The specific therapeutically effective dose level for any particular patient will depend upon a variety of factors including the arthritis being treated and the severity of the pain; activity of the specific antibody employed; the specific composition employed, the age, body weight, general health, sex and diet of the patient; the time of administration, route of administration, and rate of excretion of the specific antibody employed; the duration of the treatment; drugs used in combination or coincidental with the specific antibody employed; and like factors well known in the medical arts.

The clinician has methods available to him to measure pain in patients with arthritis pain, especially RA pain. The method and use of the invention can thus include the use of such a method to evaluate the degree of chronic pain before, during and/or after completion of treatment with the anti-netrin-1 antibody. This allows evaluating the successful completion of the treatment or the need to increase the dosage regimen or initiate an additional treatment with the antibody. One may mention the Rhumatoid Arthritis Pain Scale (RAPS) described in Diana L. Anderson, Arthritis Care & Research 45:317-323, 2001. Other methods are known under the names of visual analog pain scale (VAS), referred to above (the score must be below 20 mm, and be the lowest possible), and Medical Outcomes Study Short Form-36 Health Survey (SF-36) scale for assessing pain in patients with rheumatoid arthritis (RA) (see Wolfe F and Michaud K, 2007, J. Rheumatol 34: 8-18).

Thus, in an embodiment, the method or use combine treating chronic pain with an antibody or composition as disclosed herein, and evaluating the degree of chronic pain during and/or after completion of said treatment. This evaluation may be made using the above-described evaluation methods, for example said VAS. A VAS score below 20 mm is evidence that the treatment has reduced pain in the treated patient. The less the VAS score is below 20 mm, the less the remaining pain in said patient. If needed, e.g. the VAS score is still not below 20 mm or still close to 20 mm, the treatment with antibody is continued, or the dose regimen is increased, or one begin another phase of treatment with the antibody.

Thus in an embodiment, the method or use combine (1) determining in an arthtritis patient whether or not he is in the chronic pain phase, if the answer is yes, (2) treating chronic pain with an antibody or composition as disclosed herein, and (3) evaluating the degree of chronic pain during and/or after completion of said treatment. Determining (1) may be made using the above-described evaluation methods, for example CRP level or DAS28, possibly coupled therebetween and/or with VAS. For example, (1) allows selecting patients having chronic pain if they have lowered DAS28, and/or low CRP, especially a CRP equal or below 10 mg/liter, and/or a visual analogue scale equal or more than 20mm. Evaluation (3) may be made using the above-described evaluation methods, for example said VAS. A VAS score below 20 mm is evidence that the treatment has reduced pain in the treated patient. The less the VAS score is below 20 mm, the less the remaining pain in said patient. If needed, e.g. the VAS score is still not below 20 mm or still close to 20 mm, the treatment with antibody is continued, or the dose regimen is increased, or one begin another phase of treatment with the antibody.

In a further embodiment, the antibody of the invention is administered repeatedly according to a protocol that depends on the patient to be treated (age, weight, treatment history, etc.), and/or the response of the patient to a current treatment, which can be determined by a skilled physician.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable vehicle (or carrier or excipient) refers to a non-toxic solid, semi-solid or liquid filler, diluent, encapsulating material or formulation auxiliary of any type.

The form of the pharmaceutical compositions including the antibody of the invention and the route of administration naturally depend upon the condition to be treated, the severity of the illness, the age, weight, and gender of the patient, etc.

The antibody of the invention can be formulated for a topical, oral, parenteral, intranasal, intravenous, intramuscular, subcutaneous or intraocular administration and the like. In a particular embodiment, the antibody of the invention is administered intravenously.

In particular, the pharmaceutical compositions including the antibody of the invention may contain vehicles, which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

The vehicle or carrier can be a solvent or dispersion medium containing, for example, water, ethanol, polyol (for example, glycerol, propylene glycol, and liquid polyethylene glycol, and the like) and suitable mixtures thereof. The proper fluidity can be maintained, for example, by the use of a coating, such as lecithin, by the maintenance of the required particle size in the case of dispersion and by the use of surfactants, stabilizing agents, cryoprotectants or antioxidants. The prevention of the action of microorganisms can be brought about by antibacterial and antifungal agents. In many cases, it will be preferable to include isotonic agents, for example, sugars or sodium chloride.

Sterile injectable solutions are prepared by incorporating the active compounds in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 mL of isotonic NaCl solution and either added to 1000 mL of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

**Table 2: Description of the sequences:**

| SEQ ID NO: | Description | Sequence |
|---|---|---|
| 1 | NTN1 amino acid (aa) sequence (seq.) with signal peptide in bold and linear epitope mapping in bold and underlined | MMRAVWEALAALAAVACLVGAVRGGPGLSMFAGQ AAQPDPCSDENGHPRRCIPDFVNAAFGKDVRVSSTC GRPPARYCVVSERGEERLRSCHLCNASDPKKAHPPA FLTDLNNPHNLTCWQSENYLQFPHNVTLTLSLGKKFE VTYVSLQFCSPRPESMAIYKSMDYGRTWVPFQFYST QCRKMYNRPHRAPITKQNEQEAVCTDSHTDMRPLSG GLIAFSTLDGRPSAHDFDNSPVLQDWVTATDIRVAFS RLHTFGDENEDDSELARDSYFYAVSDLQVGGRCKCN GHAARCVRDRDDSLVCDCRHNTAGPECDRCKPFHY DRPWQRATAREANECVACNCNLHARRCRFNMELYK LSGRKSGGVCLNCRHNTAGRHCHYCKEGYYRDMG KPITHRKACKACDCHPVGAAGKTCNQTTGQCPCKDG VTGITCNRCAKGYQQSRSPIAPCIKIPVAPPTTAASSV EEPEDCDSYCKASKGKLKINMKKYCKKDYAVQIHILKA DKAGDWWKFTVNIISVYKQGTSRIRRGDQSLWIRSRD |
| | | IACKCPKIKPLKKYLLLGNAEDSPDQSGIVADKSSLVIQ WRDTWARRLRKFQQREKKGKCKKA |
| 2 | NTN1 nucleic acid seq. | ATGATGCGCGCAGTGTGGGAGGCGCTGGCGGCGC TGGCGGCGGTGGCGTGCCTGGTGGGCGCGGTGC GCGGCGGGCCCGGGCTCAGCATGTTCGCGGGCCA GGCGGCGCAGCCCGATCCCTGCTCGGACGAGAAC GGCCACCCGCGCCGCTGCATCCCGGACTTTGTCA ATGCGGCCTTCGGCAAGGACGTGCGCGTGTCCAG CACCTGCGGCCGGCCCCCGGCGCGCTACTGCGTG GTGAGCGAGCGCGGCGAGGAGCGGCTGCGCTCG TGCCACCTCTGCAACGCGTCCGACCCCAAGAAGG CGCACCCGCCCGCCTTCCTCACCGACCTCAACAAC CCGCACAACCTGACGTGCTGGCAGTCCGAGAACTA CCTGCAGTTCCCGCACAACGTCACGCTCACACTGT CCCTCGGCAAGAAGTTCGAAGTGACCTACGTGAGC CTGCAGTTCTGCTCGCCGCGGCCCGAGTCCATGG CCATCTACAAGTCCATGGACTACGGGCGCACGTGG GTGCCCTTCCAGTTCTACTCCACGCAGTGCCGCAA GATGTACAACCGGCCGCACCGCGCGCCCATCACC AAGCAGAACGAGCAGGAGGCCGTGTGCACCGACT CGCACACCGACATGCGCCCGCTCTCGGGCGGCCT CATCGCCTTCAGCACGCTGGACGGGCGGCCCTCG GCGCACGACTTCGACAACTCGCCCGTGCTGCAGG ACTGGGTCACGGCCACAGACATCCGCGTGGCCTT CAGCCGCCTGCACACGTTCGGCGACGAGAACGAG GACGACTCGGAGCTGGCGCGCGACTCGTACTTCTA CGCGGTGTCCGACCTGCAGGTGGGCGGCCGGTGC AAGTGCAACGGCCACGCGGCCCGCTGCGTGCGCG ACCGCGACGACAGCCTGGTGTGCGACTGCAGGCA CAACACGGCCGGCCCGGAGTGCGACCGCTGCAAG CCCTTCCACTACGACCGGCCCTGGCAGCGCGCCA CAGCCCGCGAAGCCAACGAGTGCGTGGCCTGTAA CTGCAACCTGCATGCCCGGCGCTGCCGCTTCAACA TGGAGCTCTACAAGCTTTCGGGGCGCAAGAGCGG AGGTGTCTGCCTCAACTGTCGCCACAACACCGCCG GCCGCCACTGCCATTACTGCAAGGAGGGCTACTAC |
| | | CGCGACATGGGCAAGCCCATCACCCACCGGAAGG CCTGCAAAGCCTGTGATTGCCACCCTGTGGGTGCT GCTGGCAAAACCTGCAACCAAACCACCGGCCAGTG TCCCTGCAAGGACGGCGTGACGGGTATCACCTGC AACCGCTGCGCCAAAGGCTACCAGCAGAGCCGCT CTCCCATCGCCCCCTGCATAAAGATCCCTGTAGCG CCGCCGACGACTGCAGCCAGCAGCGTGGAGGAGC CTGAAGACTGCGATTCCTACTGCAAGGCCTCCAAG GGGAAGCTGAAGATTAACATGAAAAAGTACTGCAA GAAGGACTATGCCGTCCAGATCCACATCCTGAAGG CGGACAAGGCGGGGGACTGGTGGAAGTTCACGGT GAACATCATCTCCGTGTATAAGCAGGGCACGAGCC GCATCCGCCGCGGTGACCAGAGCCTGTGGATCCG CTCGCGGGACATCGCCTGCAAGTGTCCCAAAATCA AGCCCCTCAAGAAGTACCTGCTGCTGGGCAACGC GGAGGACTCTCCGGACCAGAGCGGCATCGTGGCC GATAAAAGCAGCCTGGTGATCCAGTGGCGGGACA CGTGGGCGCGGCGGCTGCGCAAGTTCCAGCAGCG TGAGAAGAAGGGCAAGTGCAAGAAGGCCTAGCG |
| 3 | NTN1 aa epitopic seq. | VACNCNLHARRCRFNMELYKLSGRKSGGVCLNCRH NTAGRHCH |
| 4 | NTN1 epitopic cDNA seq. | GTGGCCTGTAACTGCAACCTGCATGCCCGGCGCT GCCGCTTCAACATGGAGCTCTACAAGCTTTCGGGG CGCAAGAGCGGAGGTGTCTGCCTCAACTGTCGCC ACAACACCGCCGGCCGCCACTGCCAT |
| 5 | aa seq. of CDR1-H (IMGT) | GYTFTSYN |
| 6 | aa seq. of CDR2-H (IMGT) | IYPGNGDT |
| 7 | aa seq. of CDR3-H (IMGT) | ARGGTGFAY |
| 8 | aa seq. of CDR1-L (IMGT) | QSVSND |
| - | aa seq. of CDR2-L (IMGT) | YAS |
| 9 | aa seq. of CDR3-L (IMGT et Kabat) | QQDYSSPWT |
| 10 | aa sequence of mouse 4C11 VH | QAYLQQSGAELVRPGASVKMSCKASGYTFTSYNMH WVKQTPRQGLEWIGAIYPGNGDTSYNQKFKGKATLT VDKSSSTAYMQLSSLTSEDSAVYFCARGGTGFAYW GQGTLVTVSA |
| 11 | aa sequence of mouse 4C11 VL | SIVMTQTPKFLLVSAGDRVTITCKASQSVSNDVAWYQ QKPGQSPKLLIYYASNRYTGVPDRFTGSGYGTDFTFT ISTVQAEDLAVYFCQQDYSSPWTFGGGTKLEIK |
| 12 | Full aa sequence of 4C11 (VH + mouse IgG1 CH) | QAYLQQSGAELVRPGASVKMSCKASGYTFTSYNMH WVKQTPRQGLEWIGAIYPGNGDTSYNQKFKGKATLT VDKSSSTAYMQLSSLTSEDSAVYFCARGGTGFAYW GQGTLVTVSAAKTTPPSVYPLAPGSAAQTNSMVTLG CLVKGYFPEPVTVTWNSGSLSSGVHTFPAVLESDLYT LSSSVTVPSSPRPSETVTCNVAHPASSTKVDKKIVPR DCGCKPCICTVPEVSSVFIFPPKPKDVLTITLTPKVTCV VVDISKDDPEVQFSWFVDDVEVHTAQTQPREEQFNS TFRSVSELPIMHQDWLNGKEFKCRVNSAAFPAPIEKTI SKTKGRPKAPQVYTIPPPKEQMAKDKVSLTCMITDFF PEDITVEWQWNGQPAENYKNTQPIMNTNGSYFVYSK LNVQKSNWEAGNTFTCSVLHEGLHNHHTEKSLSHSP GK |
| 13 | Full aa sequence of 4C11 (VL + mouse Kappa CL) | SIVMTQTPKFLLVSAGDRVTITCKASQSVSNDVAWYQ QKPGQSPKLLIYYASNRYTGVPDRFTGSGYGTDFTFT ISTVQAEDLAVYFCQQDYSSPWTFGGGTKLEIKRADA APTVSIFPPSSEQLTSGGASVVCFLNNFYPKDINVKW KIDGSERQNGVLNSWTDQDSKDSTYSMSSTLTLTKD EYERHNSYTCEATHKTSTSPIVKSFNRNEC |
| 14 | VL aa sequence of humanized variant of 4C11 | EIVMTQSPATLSVSPGERATLSCKASQSVSNDVAWY QQKPGKAPKLLIYYASNRYTGIPPRFSGSGYGTDFTL TINNIESEDAAYYFCQQDYSSPWTFGQG |
| 15 | VL aa sequence of humanized variant of 4C11 | DIQMTQSPSSLSASVGDRVTITCKASQSVSNDVAWF |
| | | QQRPGQSPRRLIYYASNRYTGVPSRFSGSGSGTDFT FTISSLEAEDAATYYCQQDYSSPWTFGQG |
| 16 | VL aa sequence of humanized variant of 4C11 | DIQMTQSPSSLSASVGDRVTITCKASQSVSNDVAWY QQKPGQAPRLLIYYASNRYTGIPPRFSGSGYGTDFTL TINNIESEDAAYYFCQQDYSSPWTFGQG |
| 17 | VL aa sequence of humanized variant of 4C11 | DIQMTQSPSSLSASVGDRVTITCKASQSVSNDVAWYL QKPGQSPQLLIYYASNRYTGVPSRFSGSGSGTDFTF TISSLEAEDAATYYCQQDYSSPWTFGQG |
| 18 | VL aa sequence of humanized variant of 4C11 | DIVMTQTPLSLPVTPGEPASISCKASQSVSNDVAWYQ QKPGQAPRLLIYYASNRYTGIPPRFSGSGYGTDFTLTI NNIESEDAAYYFCQQDYSSPWTFGQG |
| 19 | VL aa sequence of humanized variant of 4C11 | EIVMTQSPATLSVSPGERATLSCRASQSVSNDVAWY QQKPGQAPRLLIYYASNRYTGIPARFSGSGSGTEFTL TISSLQSEDFAVYYCQQDYSSPWTFGQG |
| 20 | VH aa sequence of humanized variant of 4C11 | QVQLVQSGAEVKKPGASVKVSCKASGYTFTSYNMH WVRQATGQGLEWMGAIYPGNGDTSYNQKFKGRVTI TADKSTSTAYMELSSLRSEDTAVYYCARGGTGFAYW GQG |
| 21 | VH aa sequence of humanized variant of 4C11 | QVQLQQSGPGLVKPSQTLSLTCAISGYTFTSYNMHWI RQPPGKGLEWIGAIYPGNGDTSYNQKFKGRVTITADK STSTAYMELSSLRSEDTAVYYCARGGTGFAYWGQG |
| 22 | VH aa sequence of humanized variant of 4C11 | QVQLQQSGPGLVKPSQTLSLTCAISGYTFTSYNMHW VRQATGQGLEWMGAIYPGNGDTSYNQKFKGRLTISK DTSKNQVVLTMTNMDPVDTATYYCARGGTGFAYWG QG |
| 23 | VH aa sequence of humanized variant of 4C11 | EVQLVQSGAEVKKPGESLRISCKGSGYTFTSYNMHW VRQATGQGLEWMGAIYPGNGDTSYNQKFKGRFTISR DDSKNTAYLQMNSLKTEDTAVYYCARGGTGFAYWG QG |
| 24 | VH aa sequence of humanized variant of 4C11 | QVQLQESGPGLVKPSQTLSLTCTVSGYTFTSYNMHW VRQAPGQGLEWMGAIYPGNGDTSYNQKFKGRVTISV DTSKNQFSLKLSSVTAADTAVYYCARGGTGFAYWGQ G |
| 25 | VH aa sequence of humanized variant of 4C11 | QVQLVQSGAEVKKPGASVKVSCKASGYTFTSYNMH WVRQATGQGLEWMGAIYPGNGDTSYNQKFKGRVTI |
| | | TADKSTSTAYMELSSLRSEDTAVYYCARGGTGFAYW GQG |
| 26 | VH aa sequence of humanized variant of 4C11 | QVQLQQSGPGLVKPSQTLSLTCAISGYTFTSYNMHW VRQATGQGLEWMGAIYPGNGDTSYNQKFKGRVTITA DKSTSTAYMELSSLRSEDTAVYYCARGGTGFAYWG QG |
| 27 | VH aa sequence of humanized variant of 4C11 | QVQLVQSGAEVKKPGASVKVSCKASGYTFTSYNMH WVRQAPGQGLEWMGAIYPGNGDTSYAQKFKGRVTM TRDTSTSTVYMELSSLRSEDTAVYYCARGGTGFAYW GQ |
| 28 | aa seq. of CDR1-H (Kabat) | SYNMH |
| 29 | aa seq. of CDR2-H (Kabat) | AIYPGNGDTSYNQKFKG |
| 30 | aa seq. of CDR3-H (Kabat) | GGTGFAY |
| 31 | aa seq. of CDR1-L (Kabat) | KASQSVSNDVA |
| 32 | aa seq. of CDR2-L (Kabat) | YASNRYT |
| 33 | NTN1 aa epitopic seq. | ARRCRFNMELYKLSGRKSGGVC |
| 34 | NTN1 epitopic cDNA seq. | GCCCGGCGCTGCCGCTTCAACATGGAGCTCTACAA GCTTTCGGGGCGCAAGAGCGGAGGTGTCTGC |

| | | |
|---|---|---|
| CDRs under IMGT are highlighted in bold in Table 1 where appropriate. | | |

### Definitions and further embodiments, variants and alternatives of the invention:

As used herein, a sequence "at least 85% identical to a reference sequence" is a sequence having, on its entire length, 85%, or more, in particular 90%, 91%, 92%, 93%, 94%, 95%, 96%, 97%, 98%, 99%, 99.5%, 99.6%, 99.7%, 99.8%, 99.9% or 100% sequence identity with the entire length of the reference sequence.

A percentage of "sequence identity" may be determined by comparing the two sequences, optimally aligned over a comparison window, wherein the portion of the polypeptide sequence in the comparison window may comprise additions or deletions (*i.e.* gaps) as compared to the reference sequence (which does not comprise additions or deletions) for optimal alignment of the two sequences. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison is conducted by global pairwise alignment, *e.g*. using the algorithm of Needleman and Wunsch (1970) J. Mol. Biol. 48: 443. The percentage of sequence identity can be readily determined for instance using the program Needle, with the BLOSUM62 matrix, and the following parameters gap-open=10, gap-extend=0.5.

In the context of the invention, a "conservative amino acid substitution" is one in which an amino acid residue is substituted by another amino acid residue having a side chain group with similar chemical properties (*e.g.*, charge or hydrophobicity). In general, a conservative amino acid substitution will not substantially change the functional properties of a protein. Examples of groups of amino acids that have side chains with similar chemical properties include 1) aliphatic side chains: glycine, alanine, valine, leucine, and isoleucine; 2) aliphatic-hydroxyl side chains: serine and threonine; 3) amide-containing side chains: asparagine and glutamine; 4) aromatic side chains: phenylalanine, tyrosine, and tryptophan; 5) basic side chains: lysine, arginine, and histidine; 6) acidic side chains: aspartic acid and glutamic acid; and 7) sulfur-containing side chains: cysteine and methionine. Conservative amino acids substitution groups are: valine-leucine-isoleucine, phenylalanine-tyrosine-tryptophan, lysine-arginine, alanine-valine, glutamate-aspartate, and asparagine-glutamine.

Throughout the instant application, the term "comprising" is to be interpreted as encompassing all specifically mentioned features as well optional, additional, unspecified ones. As used herein, the use of the term "comprising" also discloses the embodiment wherein no features other than the specifically mentioned features are present (*i.e.* "consisting of").

An "antibody" may be a natural or conventional antibody in which two heavy chains are linked to each other by disulfide bonds and each heavy chain is linked to a light chain by a disulfide bond. There are two types of light chain, lambda (λ) and kappa (κ). There are five main heavy chain classes (or isotypes) which determine the functional activity of an antibody molecule: IgM, IgD, IgG, IgA and IgE. Each chain contains distinct sequence domains. The light chain includes two domains or regions, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from non-hypervariable or framework regions (FR) influence the overall domain structure and hence the combining site.

"Complementarity Determining Regions" or "CDRs" refer to amino acid sequences which together define the binding affinity and specificity of the natural Fv region of a native immunoglobulin binding site. The light and heavy chains of an immunoglobulin each have three CDRs, designated CDR1-L, CDR2-L, CDR3-L and CDR1-H, CDR2-H, CDR3-H, respectively. A conventional antibody antigen-binding site, therefore, includes six CDRs, comprising the CDR set from each of a heavy and a light chain V region.

"Framework Regions" (FRs) refer to amino acid sequences interposed between CDRs, *i.e.* to those portions of immunoglobulin light and heavy chain variable regions that are relatively conserved among different immunoglobulins in a single species. The light and heavy chains of an immunoglobulin each have four FRs, designated FR1-L, FR2-L, FR3-L, FR4-L, and FR1-H, FR2-H, FR3-H, FR4-H, respectively.

As used herein, a "human framework region" is a framework region that is substantially identical (about 85%, or more, in particular 90%, 95%, 97%, 99% or 100%) to the framework region of a naturally occurring human antibody.

In the context of the invention, CDR/FR definition in an immunoglobulin light or heavy chain is to be determined based on Kabat or IMGT definitions.

The residues in antibody variable domains are conventionally numbered according to a system devised by Kabat et al. This system is set forth in Kabat et al., 1987, in Sequences of Proteins of Immunological Interest, US Department of Health and Human Services, NIH, USA (hereafter "Kabat et al."). This numbering system is used in the present specification. The Kabat residue designations do not always correspond directly with the linear numbering of the amino acid residues in SEQ ID sequences. The actual linear amino acid sequence may contain fewer or additional amino acids than in the strict Kabat numbering corresponding to a shortening of, or insertion into, a structural component, whether framework or complementarity determining region (CDR), of the basic variable domain structure. The correct Kabat numbering of residues may be determined for a given antibody by alignment of residues of homology in the sequence of the antibody with a "standard" Kabat numbered sequence. The CDRs of the heavy chain variable domain are located at residues 31-35B (H-CDR1), residues 50-65 (H-CDR2) and residues 95-102 (H-CDR3) according to the Kabat numbering system. The CDRs of the light chain variable domain are located at residues 24-34 (L-CDR1), residues 50-56 (L-CDR2) and residues 89-97 (L-CDR3) according to the Kabat numbering system. (http://www.bioinf.org.uk/abs/#cdrdef)

In the context of the invention, the amino acid residues of the antibody of the invention may be numbered according to the IMGT numbering system. The IMGT unique numbering has been defined to compare the variable domains whatever the antigen receptor, the chain type, or the species (Lefranc M.-P., "Unique database numbering system for immunogenetic analysis" Immunology Today, 18, 509 (1997) ; Lefranc M.-P., "The IMGT unique numbering for Immunoglobulins, T cell receptors and Ig-like domains" The Immunologist, 7, 132-136 (1999).; Lefranc, M.-P., Pommié, C., Ruiz, M., Giudicelli, V., Foulquier, E., Truong, L., Thouvenin-Contet, V. and Lefranc, G., "IMGT unique numbering for immunoglobulin and T cell receptor variable domains and Ig superfamily V-like domains" Dev. Comp. Immunol., 27, 55-77 (2003).). In the IMGT unique numbering, the conserved amino acids always have the same position, for instance cysteine 23, tryptophan 41, hydrophobic amino acid 89, cysteine 104, phenylalanine or tryptophan 118. The IMGT unique numbering provides a standardized delimitation of the framework regions (FR1-IMGT: positions 1 to 26, FR2-IMGT: 39 to 55, FR3-IMGT: 66 to 104 and FR4-IMGT: 118 to 128) and of the complementarity determining regions: CDR1 -IMGT: 27 to 38, CDR2-IMGT: 56 to 65 and CDR3-IMGT: 105 to 117. If the CDR3-IMGT length is less than 13 amino acids, gaps are created from the top of the loop, in the following order 111, 112, 110, 113, 109, 114, etc. If the CDR3-IMGT length is more than 13 amino acids, additional positions are created between positions 111 and 112 at the top of the CDR3-IMGT loop in the following order 112.1,111.1, 112.2, 111.2, 112.3, 111.3, etc. (http://www.imgt.org/IMGTScientificChart/Nomenclature/IMGT-FRCDRdefinition.html).

As used herein, the term "antibody" denotes conventional antibodies and fragments thereof, as well as single domain antibodies and fragments thereof, in particular variable heavy chain of single domain antibodies, and chimeric, humanized, bispecific or multispecific antibodies.

As used herein, antibody or immunoglobulin also includes "single domain antibodies" which have been more recently described and which are antibodies whose complementary determining regions are part of a single domain polypeptide. Examples of single domain antibodies include heavy chain antibodies, antibodies naturally devoid of light chains, single domain antibodies derived from conventional four-chain antibodies, engineered single domain antibodies. Single domain antibodies may be derived from any species including, but not limited to mouse, human, camel, llama, goat, rabbit and bovine. Single domain antibodies may be naturally occurring single domain antibodies known as heavy chain antibody devoid of light chains. In particular, Camelidae species, for example camel, dromedary, llama, alpaca and guanaco, produce heavy chain antibodies naturally devoid of light chain. Camelid heavy chain antibodies also lack the CH1 domain.

The variable heavy chain of these single domain antibodies devoid of light chains are known in the art as "VHH" or "nanobody". Similar to conventional VH domains, VHHs contain four FRs and three CDRs. Nanobodies have advantages over conventional antibodies: they are about ten times smaller than IgG molecules, and as a consequence properly folded functional nanobodies can be produced by in vitro expression while achieving high yield. Furthermore, nanobodies are very stable, and resistant to the action of proteases. The properties and production of nanobodies have been reviewed by Harmsen and De Haard (2007) Appl. Microbiol. Biotechnol. 77:13-22.

The term "monoclonal antibody" or "mAb" as used herein refers to an antibody molecule of a single amino acid composition that is directed against a specific antigen, and is not to be construed as requiring production of the antibody by any particular method. A monoclonal antibody may be produced by a single clone of B cells or hybridoma, but may also be recombinant, *i.e.* produced by protein engineering.

"Fragments" of (conventional) antibodies comprise a portion of an intact antibody, in particular the antigen binding region or variable region of the intact antibody. Examples of antibody fragments include Fv, Fab, F(ab')₂, Fab', dsFv, (dsFv)₂, scFv, sc(Fv)₂, diabodies, bispecific and multispecific antibodies formed from antibody fragments. A fragment of a conventional antibody may also be a single domain antibody, such as a heavy chain antibody or VHH.

The term "Fab" denotes an antibody fragment having a molecular weight of about 50,000 Da and antigen binding activity, in which about a half of the N-terminal side of H chain and the entire L chain, among fragments obtained by treating IgG with a protease, papain, are bound together through a disulfide bond.

The term "F(ab')₂" refers to an antibody fragment having a molecular weight of about 100,000 Da and antigen binding activity, which is slightly larger than the Fab bound via a disulfide bond of the hinge region, among fragments obtained by treating IgG with a protease, pepsin.

A single chain Fv ("scFv") polypeptide is a covalently linked VH::VL heterodimer which is usually expressed from a gene fusion including VH and VL encoding genes linked by a peptide-encoding linker. The human scFv fragment of the invention includes CDRs that are held in appropriate conformation, in particular by using gene recombination techniques. Divalent and multivalent antibody fragments can form either spontaneously by association of monovalent scFvs, or can be generated by coupling monovalent scFvs by a peptide linker, such as divalent sc(Fv)₂.
"dsFv" is a VH::VL heterodimer stabilized by a disulphide bond.
"(dsFv)2" denotes two dsFv coupled by a peptide linker.

The term "bispecific antibody" or "BsAb" denotes an antibody which combines the antigen-binding sites of two antibodies within a single molecule. Thus, BsAbs are able to bind two different antigens simultaneously. Genetic engineering has been used with increasing frequency to design, modify, and produce antibodies or antibody derivatives with a desired set of binding properties and effector functions as described for instance in EP 2 050 764 A1.

The term "multispecific antibody" denotes an antibody which combines the antigen-binding sites of two or more antibodies within a single molecule.

The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites.

In a particular embodiment, the epitope-binding fragment is selected from the group consisting of Fv, Fab, F(ab')₂, Fab', dsFv, (dsFv)₂, scFv, sc(Fv)₂, diabodies and VHH.

A "chimeric antibody", as used herein, is an antibody in which the constant region, or a portion thereof, is altered, replaced, or exchanged, so that the variable region is linked to a constant region of a different species, or belonging to another antibody class or subclass. "Chimeric antibody" also refers to an antibody in which the variable region, or a portion thereof, is altered, replaced, or exchanged, so that the constant region is linked to a variable region of a different species, or belonging to another antibody class or subclass.

The term "humanized antibody" refers to an antibody which is initially wholly or partially of non-human origin and which has been modified to replace certain amino acids, in particular in the framework regions of the heavy and light chains, in order to avoid or minimize an immune response in humans. The constant domains of a humanized antibody are most of the time human CH and CL domains. In an embodiment, a humanized antibody has constant domains of human origin. As used herein, the term "humanized antibody" refers to a chimeric antibody which contain minimal sequence derived from non-human immunoglobulin, e.g. the CDRs.

The term "antibody" is used to encompass all these kinds of antibodies, fragments or combination thereof.

The goal of humanization is a reduction in the immunogenicity of a xenogenic antibody, such as a murine antibody, for introduction into a human, while maintaining the full antigen binding affinity and specificity of the antibody. Humanized antibodies, or antibodies adapted for non-rejection by other mammals, may be produced using several technologies such as resurfacing and CDR grafting. As used herein, the resurfacing technology uses a combination of molecular modeling, statistical analysis and mutagenesis to alter the non-CDR surfaces of antibody variable regions to resemble the surfaces of known antibodies of the target host.

Antibodies can be humanized using a variety of other techniques including CDR-grafting (EP0239400; WO91/09967; U.S. Patent Nos. 5,530,101 and 5,585,089), veneering or resurfacing (EP0592106; EP0519596; Padlan (1991) Molecular Immunology 28(4/5):489-498; Studnicka et al. (1994) Protein Engineering 7(6):805-814; Roguska et al. (1994) Proc. Natl. Acad. Sci U.S.A. 91:969-973), and chain shuffling (U.S. Patent No. 5,565,332). Human antibodies can be made by a variety of methods known in the art including phage display methods. See also U.S. Patent Nos. 4,444,887, 4,716,111, 5,545,806, and 5,814,318; and International patent application WO98/46645, WO98/50433, WO98/24893, WO98/16654, WO96/34096, WO96/33735, and WO91/10741.

As used herein, the term "specificity" refers to the ability of an antibody to detectably bind an epitope presented on an antigen, such as netrin-1, while having relatively little detectable reactivity with non-netrin-1 proteins or structures (such as other proteins presented on cancer cells, or on other cell types). Specificity can be relatively determined by binding or competitive binding assays, using, e.g., Biacore instruments, as described elsewhere herein. Specificity can be exhibited by, e.g., an about 10:1, about 20:1, about 50:1, about 100:1, 10.000:1 or greater ratio of affinity/avidity in binding to the specific antigen versus nonspecific binding to other irrelevant molecules (in this case the specific antigen is netrin-1).

The term "affinity", as used herein, means the strength of the binding of an antibody to an epitope. The affinity of an antibody is given by the dissociation constant Kd, defined as [Ab] x [Ag] / [Ab-Ag], where [Ab-Ag] is the molar concentration of the antibody-antigen complex, [Ab] is the molar concentration of the unbound antibody and [Ag] is the molar concentration of the unbound antigen. The affinity constant Ka is defined by 1/Kd. Preferred methods for determining the affinity of mAbs can be found in Harlow, et al., Antibodies: A Laboratory Manual, Cold Spring Harbor Laboratory Press, Cold Spring Harbor, N.Y., 1988), Coligan et al., eds., Current Protocols in Immunology, Greene Publishing Assoc. and Wiley Interscience, N.Y., (1992, 1993), and Muller, Meth. Enzymol. 92:589-601 (1983), which references are entirely incorporated herein by reference. One preferred and standard method well known in the art for determining the affinity of mAbs is the use of Biacore instruments.

The present invention will now been described in more detail using the following experiments referring to the Figures.

### Description of the Figures:

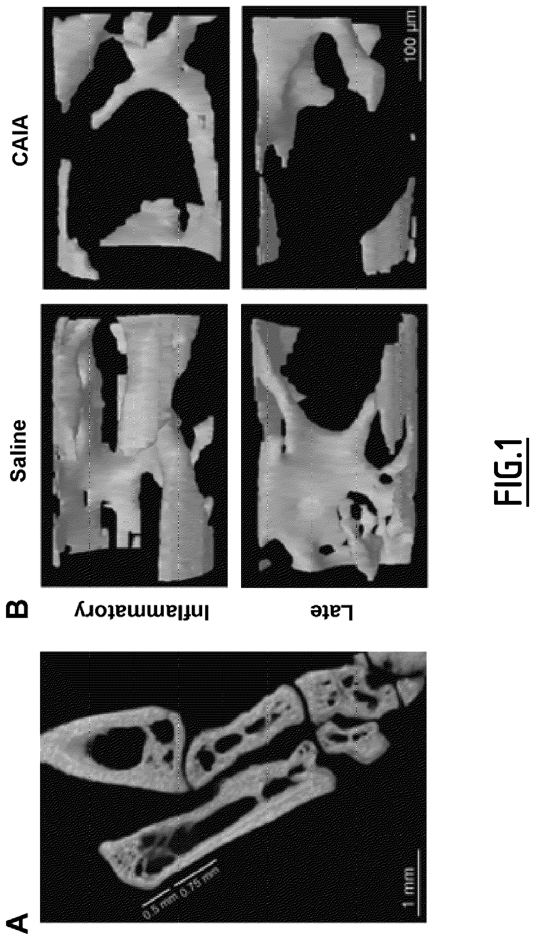

**Figure 1****:** induction of arthritis and bone loss in CAIA model.
   Collagen antibody-induced arthritis (CAIA) promotes localized bone loss in the calcaneus. Ankle joints were harvested from the inflammatory (day 17-20) and late (day 54-56) phase of the CAIA model. Representative micro-CT images of quantification area in the calcaneus (scales indicated on the image from top to bottom: 0.5 mm, 0.75 mm and 1 mm) **(A)** and images of calcaneus from saline and CAIA mice in inflammatory and late phase (scale indicated on the image: 100 µm) **(B).**

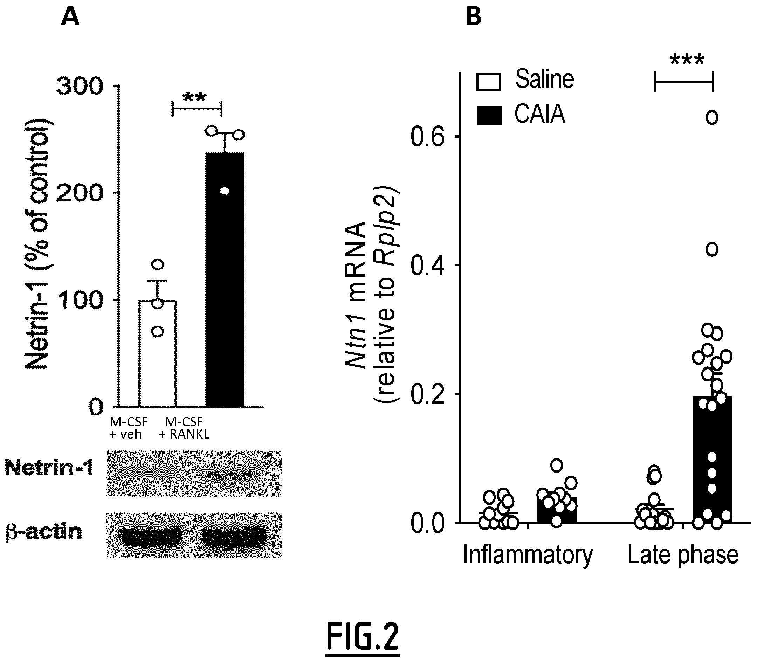

**Figure 2****.** Osteoclasts produce the axon guidance molecule netrin-1. **A.** Western Blot images and quantification of netrin-1 in day 6 primary osteoclast culture (M-CSF+RANKL) compared to monocytes (M-CSF+veh). Data presented as mean ± SEM, n=3 (cultures from 3 different mice), **p<0.01. **B.** mRNA levels in whole DRG extracts of saline control and CAIA mice (inflammatory phase: day 17-20; post-inflammatory phase: day 54-59). Data are presented as mean ± SEM, *p<0.05, **p<0.01.

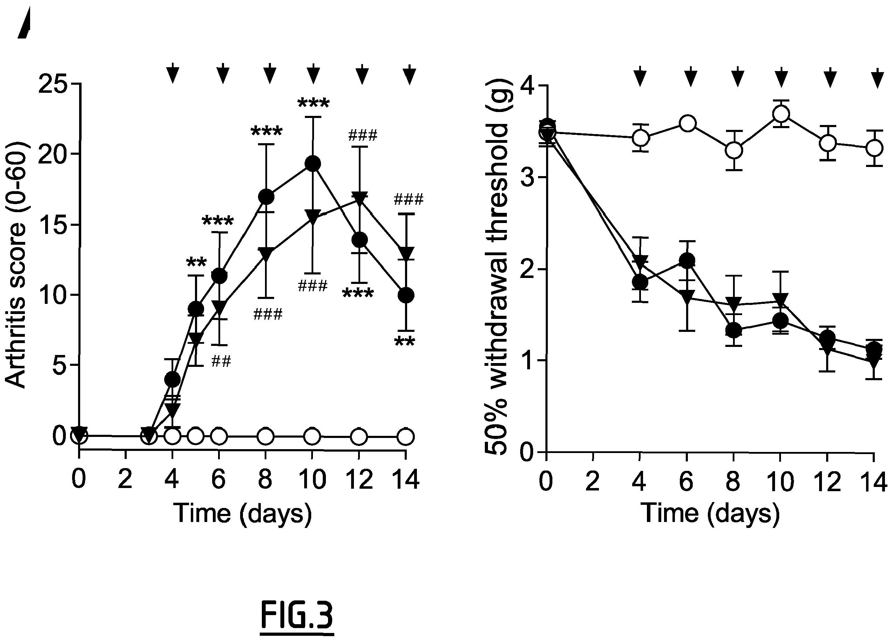

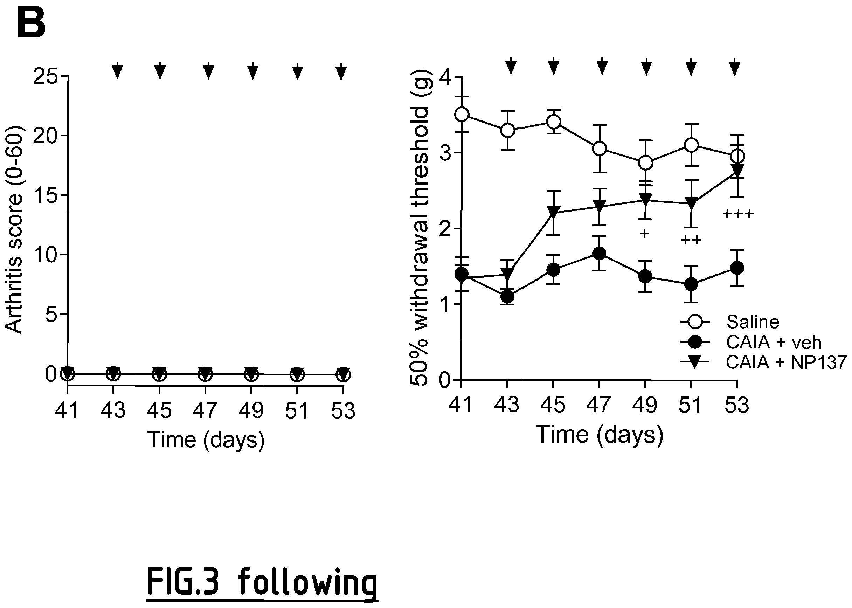

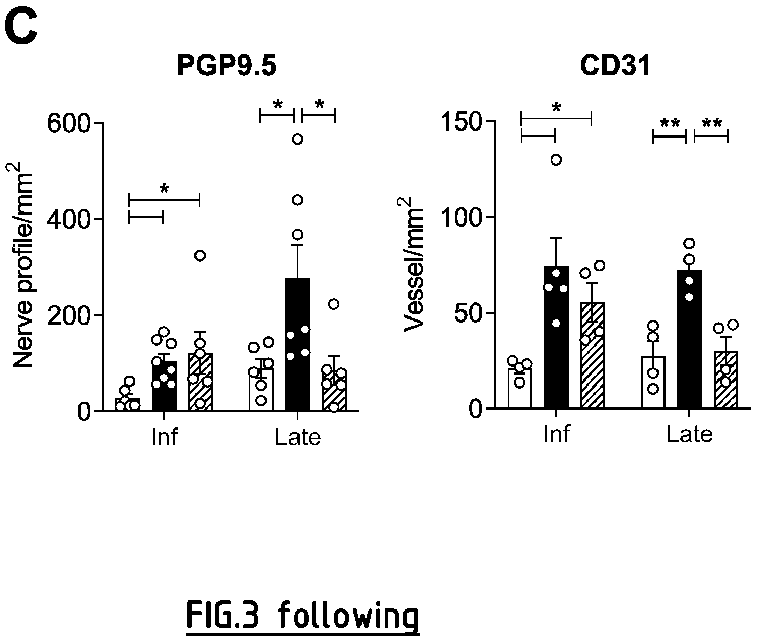

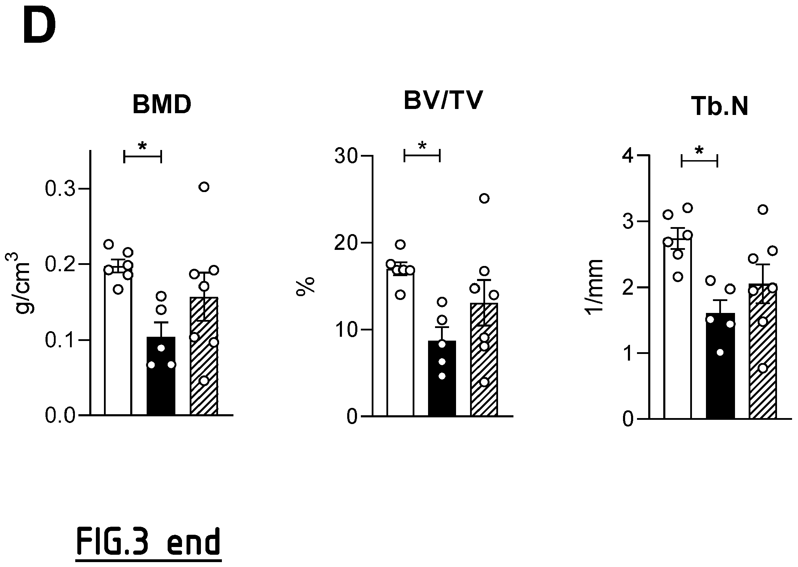

**Figure 3****.** NP-137 has anti-nociceptive properties in the CAIA model and prevents increase in innervation and vascularization in the late phase of the model.
   **A.** Inflammatory phase. No effect of NP-137 (10 mg/kg, i.p. Q.O.D day 4-14) on CAIA-induced arthritis scores of ankle joints or mechanical withdrawal thresholds. Data are presented as mean ± SEM, n=6-8 mice/group, curve legends on **Figure 3** **B,** *represents saline vs. CAIA + vehicle, # represents saline vs. CAIA + NP137.
   **B.** Post-inflammatory (late) phase. NP-137 (10 mg/kg, i.p. Q.O.D day 43-53) did not alter the arthritis scores (which had resolved by day 43) but attenuated CAIA-induced mechanical hypersensitivity. Data are presented as mean ± SEM, n=6-8 mice/group, + represents CAIA + NP137 vs. CAIA + vehicle.
   **C.** Scatter graphs show that treatment with NP-137 reduced CAIA-induced increase of nerve profile densities and blood vessel densities in the periosteum of calcaneus in the late phase of the model. White bar: saline; black bar: CAIA + vehicle; hatched bar: CAIA + NP137. Data are presented as mean ± SEM. *p<0.05, **p<0.01. Arrows indicate drug administration. Inf: inflammatory phase.
   **D.** Quantitative evaluation of trabecular bone mineral density (BMD), bone volume per tissue volume (BV/TV) and trabecular number (Tb.N) in the calcaneus of late phase of the CAIA model show that NP-137 does not exaggerate CAIA-induced bone loss, but that there may be a trend towards protection. White bar: saline; black bar: CAIA + vehicle; hatched bar: CAIA + NP137. Data are presented as mean ± SEM. *p<0.05. Arrows indicate drug administration. Inf: inflammatory phase.

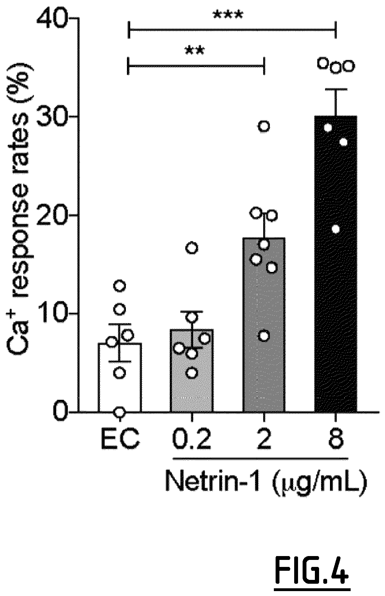

**Figure 4****:** Netrin-1 induces intracellular Ca²⁺ flux in neuronal cultures.
   Bar graphs represent intracellular [Ca²⁺] signal response rate in DRG neuronal cultures following stimulation with mouse recombinant netrin-1 protein (0.2, 2 and 8 µg/mL) or extracellular solution (EC) as control. Data are presented as mean ± SEM, n=6-7 technical replicates ^{*,+}p<0.05, ^{**,##,++}p<0.01, ^{***,###,+++}p<0.001.

### MATERIALS AND METHODS

### Animals

BALB/c female mice (10-12 weeks, 20-25 g) were purchased from Charles River and Janvier laboratories and housed in standard cages (3-5 mice per cage) in climate-controlled environment with 12 h light/dark cycle and *ad libitum* access to food and water. All experiments conformed to the ARRIVE guidelines and protocols approved by local ethical committees in Sweden (Stockholm Norra Djurförsöksetiska nämnd) and France (CEMEA-Auvergne).

### Induction and scoring of arthritis

Collagen antibody-induced arthritis (CAIA) was induced as previously described (Bas DB et al., Arthritis Rheum. 2012;64:3886-96). Briefly, mice were injected intravenously (i.v.) with anti-collagen type II (CII) arthritogenic antibody cocktail (1.5 mg/mouse, Chondrex) containing 5 monoclonal CII antibodies on day 0. Lipopolysaccharide (LPS, 25 µg/mouse, Chondrex) was injected intraperitoneally (i.p.) on day 3 or 5 to synchronize inflammation. LPS injection day 3 gives higher arthritis incidence and the protocol was therefore refined. The control group received saline i.v. on day 0 and i.p. on day 3 or 5. The degree of inflammation was assessed by visual inspection of the fore and hind paws. A score of 1 point was given for each inflamed toe or knuckle and a score of 5 points was given to inflamed wrist or ankle, resulting in maximum possible score of 15 points per paw and 60 points per mouse.

### Micro-computed tomography imaging

Mice were euthanized with decapitation under deep isoflurane anesthesia (4%). Hind ankle joints were harvested, post-fixed in 4% paraformaldehyde (PFA) for 48 h, washed with 0.1 M PBS and stored at 4°C until further analysis. Distal tibia, talus and calcaneus were analyzed using a micro-computed tomography (microCT) imaging system (Skyscan 1272, Bruker). The scanning process was conducted at 10 µm voxel size, 60 kVp/166 µA X-Ray power and 627 ms integration time. Acquired images were reconstructed using NRecon software (Bruker) and analyzed using CT analyzer program (Bruker).

### Drugs and drug delivery

Anti-Netrin-1 antibody (NP137; AB 2811180 in The Antibody Registry) (10 mg/kg) was injected i.p. every other day on day 4-14 or 43-51 of the CAIA model. Control buffer (containing 20 mM Tris, 5% sucrose and 0.01% Tween-20) was used as vehicle.

### Behavioral tests

Mechanical hypersensitivity was assessed by application of calibrated von Frey OptiHair filaments (Marstock) (Chaplan SR et al., J. Neuroscience Methods. 1994 ; 53 :55-63). A series of filaments with a logarithmically incremental stiffness of 0.5, 1, 2, 4, 8, 16 and 32 mN were applied to the plantar surface of the hind paw and held for 2-3 seconds. A brisk withdrawal of the paw was noted as a positive response. Values were calculated as 50% withdrawal thresholds (*i.e.* the thresholds of mechanical hypersensitivity at which there was a 50% probability of paw withdrawal) using the up-down method (Chaplan SR et al., supra). The experimenter was blinded to groups and treatments during the behavioral tests.

### Osteoclast cultures and analysis

Bone marrow cells obtained from mouse femur and tibia were differentiated into osteoclasts with 30 ng/mL macrophage colony-stimulating factor (M-CSF, R&D) and 50 ng/mL receptor activator of nuclear kappa-B ligand (RANKL, R&D). On day 6, cell were either fixed with 4% PFA or homogenized in lysis buffer for protein extraction. Protein extract was used to measure netrin-1 levels using Western Blot.

### Western Blot

Osteoclasts were homogenized in lysis buffer containing PBS, 1% sodium dodecyl sulphate (SDS) and a cocktail of protease inhibitors. 5 µg of proteins were separated using gel electrophoresis and transferred onto nitrocellulose membranes. Nonspecific binding sites were blocked with 5% non-fat milk, and the membranes was probed with primary antibody overnight at 4oC (netrin-1, 1:1000, Abcam ab1267729). After washing, the membranes were incubated with secondary antibodies conjugated to HRP (CST) and chemiluminescent reagent (SuperSignal West Femto, ThermoFisher) was used to visualize immunopositive band on ChemiDoc Imaging System (Biorad). Membranes were then stripped and reprobed with primary antibody as protein reference (β-actin, 1:10000, CST 3700). Quantification was performed with ImageJ, and results are presented as percentage change to the control group.

### Primary neuronal cultures and calcium imaging

DRG neuronal cultures were prepared using a modified version of previously described protocol (Malin SA et al., Nat Protoc. 2007; 2:152-60). In brief, mouse DRGs (C1-L6) were dissected and enzymatically digested with papain (60 U; 30 min at 37°C) followed by collagenase I and dispase II (12 and 14 mg/mL; 30 min at 37°C). Next, cells were triturated in Ham's F12 Nutrient Mixture medium supplemented with 10% FBS (Gibco) and 1% penicillin/streptomycin (ThermoFisher), and seeded on glass-bottomed dishes (Mattek) pre-coated with poly-D-lysine (Corning) and laminin (Sigma-Aldrich). Cells were kept in a humidified 37°C incubator with 5% CO₂ for 24 h. After 24 h, cells were loaded with the calcium indicator Fluo-3 AM (4.4 µM, ThermoFisher) for 30 min and calcium imaging was performed with Zeiss LSM800 confocal microscope. Mouse recombinant netrin-1 (0.2, 2 or 8 µg/mL, R&D) was directly applied to the cells and cells responses were recorded for 16 min followed by application of KCI (50 mM) to detect functional neurons. Images were analyzed using ImageJ.

### Calcium imaging

Cells were loaded with the calcium indicator Fluo-3 AM (4.4 µM, ThermoFisher) for 30 min at room temperature and washed with extracellular solution (EC; 10 mM Hepes, 2 mM CaCl₂, 3 mM KCI, 145 mM NaCl, 2 mM MgCl₂, 10 mM glucose, pH 7.4). Calcium imaging was then performed with Zeiss LSM800 confocal microscope with the following settings: 20x objective, argon laser (488 nm excitation), change in emission (506 nm) measured every 2.5 sec using a photomultiplier tube. Mouse recombinant netrin-1 (0.2, 2 or 8 µg/mL, R&D) was directly applied to the cells and cells responses were recorded for 16 min. EC solution was used as negative control for the experiment. At the end of each experiment, KCI (50 mM) was applied to detect functional neurons. Acquired images were analyzed using ImageJ. In each image, the mean fluorescence intensity (F) of each neuron was measured manually selecting all cells present in the field of image. The baseline recording (F₀) of each neuron was calculated as the average mean signal of the initial 10 images of the series before application of reagents. A cell was considered positive if the increase of the fluorescent signal was at least ≥25% compared to baseline. Data are presented as response rates for each dish (positive cells/total cells).

### Statistical analyses

For comparing changes over time, two-way analysis of variance was used followed by Bonferroni post hoc test. Mean differences between three groups or more were compared using one-way analysis of variance followed by Bonferroni or Dunnett post hoc test, while differences in two groups were analyzed by student's *t*-test. All tests were performed using GraphPad Prism software. p values < 0.05 were considered significant.

### RESULTS

### The capacity of osteoclasts to produce neurogenic factors

It has been documented that osteoclasts play a key role in arthritis. The well described model of CAIA injection that is associated with local inflammation and long lasting pain measured by mechanical hypersensitivity behavior tests is described in Rudjilto et al., Pain, 2021. It is first assessed here whether netrin-1 is expressed by osteoclasts in CAIA-induced arthritis. To assay that osteoclasts are capable of producing factors that modulate these processes, *in vitro* experiments were performed using primary osteoclasts. Cultured osteoclasts were exposed to vehicle (PBS), starting from day 2 for a total of 4 days. Cells cultured in the presence of both M-CSF and RANKL produced TRAP⁺ multinucleated cells on day 6, which are common characteristics of osteoclasts, as opposed to cells cultured with M-CSF only that did not generate cells with this morphology.

Netrin-1 protein levels were assessed by Western blotting of whole cell lysate harvested on day 6. Osteoclast differentiation led to significant increase in netrin-1 protein levels (Figure 3A)

Netrin-1 mRNA levels were measured in whole DRG extracts of saline control and CAIA mice (inflammatory phase: day 17-20; post-inflammatory phase: day 54-59). Osteoclast differentiation led to significant increase in netrin-1 mRNA levels (Figure 3B).

### Role of netrin-1 in nociception and efficacy of monoclonal antibody NP137

We examined the contribution of netrin-1 to CAIA-induced pain-like behavior. Systemic administration of the anti-netrin-1 antibody (NP137, 10 mg/kg, every second day) did not affect arthritis score or mechanical hypersensitivity in the inflammatory phase (Figure 3A). However, we observed a progressive reversal of CAIA-induced hypersensitivity in the late phase with NP137 treatment initiated day 43, which was statistically significant from day 49 onwards (p<0.05 versus vehicle control) (Figure 3B).

Scatter graphs (Figure 3C) show that treatment with NP137 reduced CAIA-induced increase of nerve profile densities and blood vessel densities in the periosteum of calcaneus in the late phase of the model.

Quantitative evaluation of trabecular bone mineral density (BMD), bone volume per tissue volume (BV/TV) and trabecular number (Tb.N) in the calcaneus of late phase of the CAIA model show (Figure 3D) that NP137 does not exaggerate CAIA-induced bone loss, but that there may be a trend towards protection.

To confirm the pronociceptive property of netrin-1, we performed *in vitro* calcium imaging experiments with primary DRG neurons. Netrin-1 stimulation of primary DRG neurons increased the percentage of cells that showed elevated intracellular Ca²⁺ signal in a dose-dependent fashion (Figure 4).

### DISCUSSION

We here identified the possible nociceptive roles of netrin-1 released by osteoclasts. While the exact mechanisms as to how this factor induce pain in the CAIA model remains unclear, however our findings suggest the view that in response to inflammation, osteoclasts produce netrin-1 that on a long lasting period modulates the activity of neurons involved in nociception.

These experiments show that blockade of netrin-1 using the anti-netrin-1 monoclonal antibody NP137 led to reversal of pain-like behavior. They also demonstrate that netrin-1 expression was upregulated only in the late phase of the CAIA model, suggesting that netrin-1 mediates osteoclast-induced long-lasting pain in this model. This allow proposing netrin-1 as therapeutical intervention for treating chronic pain in RA, using an anti-netrin-1 monoclonal antibody such as NP137.

### SUPPLEMENTARY INFORMATION

### ^{99m}Tc-HMDP scintigraphic imaging

To evaluate bone remodelling, ^{99m}Tc-HMDP scintigraphic imaging was performed on day 6, 17, 31 and 49 of the CAIA model. Mice were injected i.p. with ^{99m}Tc-HMDP radiotracer (10 MBq/mouse, Osteocis^{®}, IBA) and imaging was acquired 2.5 h post-injection. Planar acquisition was performed for 5 minutes (15% window at 140 keV) on the animal positioned in ventral decubitus on a parallel collimator (20mm/1.8/0.2) of a gamma camera for small animal (ylmager^{®}, Biospace) under isoflurane anaesthesia (1%). Quantitative analysis of bone scintigrams was performed with Gammavision+software (Biospace) using fixed-sized rectangular regions of interest on both hind paws. For each time point and animal, scintigraphic ratio of bone (SR_{b}) were both calculated as follow: SR = pathological paw average activity (cpm)/contralateral paw average activity (cpm).

### Immunoassay

Mice were deeply anesthetized with isoflurane (4%) and blood was collected with cardiac puncture. Blood was then allowed to clot for 30 min at room temperature, centrifuged (4800 *g* for 10 min at 4°C) and the collected serum stored at -80°C until analyses. Serum levels of C-telopeptide of type I collagen (CTX-I, bone erosion marker) and propeptide of type I procollagen (PINP, boner formation marker) were quantified in mouse serum using the RatLaps CTX-I EIA and Rat/Mouse PINP EIA kits (IDS). Samples and standards were measured in duplicates.

### Tissue processing for paraffin-embedded sections

Animals were deeply anesthetized with isoflurane (4%) and transcardially perfused with PBS followed by 4% PFA. Hind ankle joints were harvested and postfixed for 48 h in 4% PFA. Next, tissues were washed in PBS and decalcified in 10% ethylenediaminetetraacetate (EDTA) solution (Sigma-Aldrich) for 4-5 weeks. Decalcification solution was changed every week. The decalcified joints were then dehydrated using 70% ethanol and xylene followed by embedding in paraffin. Sagittal sections (5 µm) were cut using a rotary microtome (Microm HM360, Thermo Fisher) and mounted onto glass slides.

### Immunohistochemistry

Prior to staining, paraffin-embedded sections were deparaffinized, blocked for endogenous peroxidase and subjected to overnight antigen retrieval in Tris-acetate buffer (pH 6, DAKO) at 60°C. The following day, sections were blocked with 5% normal goat serum to reduce nonspecific binding and incubated with primary antibodies (cathepsin K, 1:100, OriGene TA318059; PGP9.5, 1:500, Cedarlane 184; CD31, 1:250, Abcam ab124432) for 2 h. Subsequently, alkaline phosphatase-conjugated IgG (1:500, Sigma-Aldrich) was used for secondary detection followed by visualization using liquid fast-red substrate kit (Abcam). All sections were counterstained with Mayer's hematoxylin and mounted with ProlongGold antifade mountant (ThermoFisher).

### In situ hybridization

Paraffin-embedded sections were in situ hybridized using a modified version of the RNAScope 2.0 high-definition procedure (ACD) to detect mRNA expression of Trap (probe-Mm-Acp5). In short, sections were incubated with pretreatment 1 for 10 min at room temperature, pretreatment 2 for 15 min at 85°C and pretreatment 3 was replaced with a pepsin treatment (10% diluted in RNase-free water, DAKO) for 20 min at 40°C. The subsequent hybridization and amplification steps were performed according to the manufacturer's instructions. Next, sections were incubated with digoxigenin (DIG)-labeled tyramide (1 :200) from TSA plus DIG reagent kit (PerkinElmer) for 5 min at room temperature followed by labelling with alkaline phosphatase-conjugated anti-DIG FAB fragments (1:1000, Roche). Labelled sections were visualized with liquid permanent red (DAKO), counterstained with Mayer's hematoxylin and mounted with Aquatex (Sigma-Aldrich).

### Histomorphometry

Sections were scanned using a NanoZoomer Slide Scanner (Hamamatsu Photonics) and images were analyzed using NDP.view2 software (Hamamatsu Photonics). The conventional histomorphometric parameter osteoclast surface per bone surface (Oc.S/BS) was estimated using a grid of sinus curves. CTSK- and Trap-stained cells were quantified at intersection points between sinus curves and bone surfaces. Bone vascular and nerve densities were estimated by quantifying CD31- and PGP9.5-stained vessels or nerves in areas located below 100 µm from bone surfaces. A point-grid was used to estimate the size of the tissues and local vessel and nerve densities were expressed as the number of vessels or nerves per tissue area (mm²). Analysis was performed by investigators who were blinded to the group assignments.

### Quantitative real-time polymerase chain reaction

Mice were euthanized with decapitation under deep isoflurane anesthesia (4%). Hind ankle joints and L3-L5 dorsal root ganglia (DRGs) were harvested, flash-frozen and stored at -80°C until further analysis. mRNA from these tissues was extracted using TRIzol reagent (Invitrogen) according to the manufacturer's protocol followed by cDNA synthesis using MultiScribe Reverse Transcriptase (Invitrogen). qPCR was performed using StepOne Real-Time PCR Systems (Applied Biosystems) using pre-developed Taqman primer/probe for *Ntn1* (Mm00500896_m1), Threshold cycle values for each sample were used to calculate the number of cell equivalents in the test samples using the standard curve method. The data were normalized to mRNA levels of housekeeping genes (*Hprt1* or *Rplp2*) and expressed as relative expression.

### Bone marrow-derived primary osteoclast culture

Bone marrow was flushed from mouse tibia and femur using 27-gauge needle, and cells were isolated by centrifugation at 300*g* for 5 min. Cells were then cultured in Eagle's Minimum Essential Medium - alpha modification (αMEM, Gibco) supplemented with 10% fetal bovine serum (FBS, Sigma-Aldrich), 1% penicillin/streptomycin (ThermoFisher), 30 ng/mL macrophage colony-stimulating factor (M-CSF, R&D) and 50 ng/mL receptor activator of nuclear kappa-B ligand (RANKL, R&D) at a density of 1.25 × 10⁵ cells/well for 6 days in a 37°C incubator with 5% CO₂ Cells cultured without RANKL, which prevents osteoclast differentiation and would instead yield monocytes/macrophages, were included as control. Medium was refreshed every 2 days and starting on day 2 cells were stimulated with either 600 nM zoledronate (Sigma-Aldrich), 1 µM T06 or vehicle (PBS). On the day of medium change, culture supernatant was collected and stored at -80°C for further analysis. At the end of experiment, cells were fixed in 4% PFA for 5 min and stained for tartrate resistant acid phosphatase (TRAP) using leukocyte acid phosphatase kit (Sigma-Aldrich). In short, fixed cells were incubated with the following reagents: Fast Garnet GBC, sodium nitrite, naphthol AS-BI phosphoric acid, tartrate and acetate solution for 30-45 min at 37°C. Quantification of multinucleated cells (>3 nuclei) was performed in three independent experiments using a brightfield microscope (Nikon Eclipse TE-200).

## Claims

1. An anti-netrin-1 antibody or an antigen-binding fragment thereof, for use as an analgesic or antinociceptive medicament to treat Rheumatoid arthritis-associated pain in the chronic pain phase.

2. The anti-netrin-1 antibody or an antigen-binding fragment thereof for the use of claim 1, wherein the antibody or antigen-binding fragment specifically binds to a polypeptide of sequence SEQ ID NO: 33.

3. The anti-netrin-1 antibody or an antigen-binding fragment thereof for the use of claim 1 or 2, wherein the antibody is a monoclonal antibody or an antigen-binding fragment thereof, wherein the antibody or fragment comprises
a variable domain VH comprising:
- a H-CDR1 having a sequence set forth as SEQ ID NO: 5;
- a H-CDR2 having a sequence set forth as SEQ ID NO: 6;
- a H-CDR3 having a sequence set forth as SEQ ID NO: 7;
a variable domain VL comprising:
- a L-CDR1 having a sequence set forth as SEQ ID NO: 8;
- a L-CDR2 having a sequence YAS;
- a L-CDR3 having a sequence set forth as SEQ ID NO: 9;
or
a variable domain VH comprising:
- a H-CDR1 having a sequence set forth as SEQ ID NO: 28;
- a H-CDR2 having a sequence set forth as SEQ ID NO: 29;
- a H-CDR3 having a sequence set forth as SEQ ID NO: 30;
a variable domain VL comprising:
- a L-CDR1 having a sequence set forth as SEQ ID NO: 31;
- a L-CDR2 having a sequence set forth as SEQ ID NO: 32;
- a L-CDR3 having a sequence set forth as SEQ ID NO: 9.

4. The anti-netrin-1 antibody or an antigen-binding fragment thereof for the use of any one of claims 1 to 3, wherein the antibody is a monoclonal antibody or an antigen-binding fragment thereof, and the antibody or fragment thereof comprises a pair of VH and VL sequences selected from the following pairs: SEQ ID NO: 27 and 19, SEQ ID NO: 20 and 14, SEQ ID NO: 21 and 15, SEQ ID NO: 22 and 16, SEQ ID NO: 23 and 17, SEQ ID NO: 24 and 17, SEQ ID NO: 25 and 16, SEQ ID NO: 26 and 17, SEQ ID NO: 22 and 17, SEQ ID NO: 25 and 18, SEQ ID NO: 21 and 16, and preferably the antibody or fragment thereof comprises a pair of VH and VL sequences SEQ ID NO: 22 and 16.

5. The anti-netrin-1 antibody for the use of any one of claims 1 to 4, comprising a Human IgG1 Constant heavy chain (CH) and/or a Human IgG1 Constant light chain (CL), in particular a human kappa constant domain.

6. The antibody for the use of any one of claims 1 to 5, comprising a VH of sequence of SEQ ID NO: 22 and a VL of sequence SEQ ID NO: 16.

7. The antibody for the use of to claim 6, further comprising a human IgG1 CH Genbank AEL33691.1 modified R97K, and a human IgG1 CL Kappa Genbank CAC20459.1.

8. A pharmaceutical composition comprising an antibody according to any one of claims 1 to 7, for use an analgesic or antinociceptive medicament to treat Rheumatoid arthritis-associated pain in the chronic pain phase.
